# EUROPÄISCHE PATENTSCHRIFT

(11) **EP 1 941 837 B1**
(45) Veröffentlichungstag und Bekanntmachung des Hinweises auf die Patenterteilung: **11.01.2012**
(21) Anmeldenummer: 07024880.2
(22) Anmeldetag: 21.12.2007
(51) Int. Cl.: A61B 17/08

(54) **Vorrichtung zur atraumatischen Versorgung von Wunden**
Device for non-traumatic caring for wounds
Dispositif d'alimentation atraumatique de plaies

(30) Priorität: 08.01.2007 DE 102007001278
(43) Veröffentlichungstag der Anmeldung: 09.07.2008
(73) Patentinhaber: Kaessmann, Hans-Jürgen, 23570 Travemünde (DE)
(72) Erfinder: Kaessmann, Hans-Jürgen, 23570 Travemünde (DE); Kaessmann, Uwe, 23795 Bad Segeberg (DE)
(74) Vertreter: Hauck Patent- und Rechtsanwälte

(56) Entgegenhaltungen:
- DE-A1- 3 444 782
- DE-A1-102004 059 499
- DE-C- 618 393
- US-A- 2 012 755

## Beschreibung

Die vorliegende Erfindung betrifft eine Vorrichtung zur atraumatischen, nichtinvasiven Versorgung von Wunden. Bei den zu versorgenden Wunden kann es sich um Operations- und Gelegenheitswunden handeln. Die Vorrichtung dient als Wundverschluß und als Wundabdeckung.

Aus dem Stand der Technik sind verschiedene Systeme von Wundreißverschlüssen bekannt, die zwei ineinander greifende und jeweils an einem Tragband befestigte Verschlußgliederreihen, einen Schieber und an den Tragbändern befestigtes Abstandspolster aufweisen.

DE 44 00 732 A1 beschreibt einen Wundreißverschluß mit großflächig ausgebildeten Tragbändern. Die Tragbänder bestehen aus einem festen Material und sind in ihrer Längsrichtung entlang einer Faltkante zu einem Doppeltragbandbereich gefaltet. An den Tragbändern ist ein Abstandspolster vorgesehen, das einen Wundfreiraum bildet. Nachteilig an der beschriebenen Konstruktion ist, daß aus der Wunde abgegebene Körperflüssigkeit mit den Verschlussgliederreihen in Berührung kommen und diese verkleben können. Hierdurch kann eine Blockade des Wundreißverschlusses auftreten.

Eine weitere Konstruktion eines Wundreißverschlusses ist in DE 37 06 599 C2 sowie EP 0 271 741 B 1 beschrieben. Der bekannte Wundreißverschluß besteht ebenfalls aus Tragbändern und daran befestigten Verschlußreihen, die über Abstandspolster von der Wunde beabstandet sind. Auch bei diesem Wundreißverschluß kann ausgetretene Wundflüssigkeit in den Reißverschluß gelangen und diesen blockieren.

Aus US 3,863,640 ist ein Wundverschluß bekannt, der einen wiederverschließbaren Gleitverschluß besitzt. Die Unterseite des Gleitverschlusses ist mit einer Klebeschicht versehen. Ferner ist bekannt, im zentralen Bereich des Wundverschlusses eine Gazeschicht oder eine andere absorbierende Wundauflage vorzusehen. Auch bei diesem Wundverschluß besteht die Gefahr, daß austretende Wundflüssigkeit den wiederverschließbaren Gleitverschluß zusetzt und verklebt.

Aus DE 61 83 93 ist ein lösbarer Wundverband bekannt, der mit einem Reißverschluss geöffnet werden kann.

Aus US 2,012,755 ist ein Wundverband mit einem Reißverschluss bekannt, bei dem ein selbstklebendes Pflaster mit einem elastischen Streifen vernäht ist, auf dem ein Reißverschluss gehalten ist. Die Verschlussgliederreihen des Reißverschlusses sind im Wundbereich mit einem vorstehenden elastischen Streifen abgedeckt.

Aus DE 34 44 782 ist ein temporärer Wundverschluss mit einem Gleitschieber bekannt. Der Gleitschieber ist zur Wunde hin mit einem Untertrittstreifen abgedeckt.

Aus DE 10 2004 059 499 A1 (Basis für den Oberbegriff des Anspruchs 1) ist ein Wundreißverschluss mit einem semiresorptiven Abstandspolster bekannt. Die Abstandspolster weisen eine Trennschicht auf, die einen Flüssigkeitsdurchtritt durch eine Resorptionsschicht des Abstandspolsters verhindert.

Technische Aufgabe der vorliegenden Erfindung ist es, eine Vorrichtung zur atraumatischen Versorgung einer Wunde bereitzustellen, die durch Blut- oder Wundflüssigkeit in ihrer Funktionsweise nicht beeinträchtigt werden und gleichzeitig als saubere und zuverlässige Wundabdeckung Verwendung finden kann.

Erfindungsgemäß wird die Aufgabe durch einen Wundreißverschluß mit den Merkmalen aus Anspruch 1 gelöst. Vorteilhafte Weiterführungen bilden die Gegenstände der Unteransprüche.

Die erfindungsgemäße Vorrichtung dient zur atraumatischen Versorgung von Wunden, die sowohl einen Verschluß einer offenen Wunde als auch eine Wundabdeckung der Wunde bietet. Die Vorrichtung besteht aus zwei Tragbändern, an denen jeweils eine Verschlußgliederreihe befestigt ist. Die Verschlußgliederreihe kann ein Reißverschluß, ein Gleitverschluß oder ein sonstiger wiederverschließbarer Verschluß sein. Die erfindungsgemäße Vorrichtung besitzt ferner Abstandspolster, die jeweils an dem Tragband vorgesehen sind. Das Abstandspolster erfüllt zwei Aufgaben. Einerseits dient es als Wundauflage und kann austretende Flüssigkeit aus der Wunde aufnehmen. Andererseits dient das Abstandspolster dazu, den Abstand zwischen den Verschlußgliederreihen und der Wunde sicherzustellen. Erfindungsgemäß ist an mindestens einem Tragband des Verbandes zwischen dem Abstandspolster und der Verschlußgliederkette ein flüssigkeitsundurchlässiges Folienband vorgesehen. Erfindungsgemäß ist bei jedem Tragband zwischen dem Abstandspolster und der Verschlussgliederreihe jeweils ein flüssigkeitsundurchlässiges Folienband vorgesehen, das im geschlossenen Zustand der Verschlussgliederreihen diese abdecken und einander überlappen, wobei eines der Folienbänder weiter über das Abstandspolster vorsteht als das gegenüberliegende Folienband und die Folienbänder eine überlappende Abdichtung bilden, bei der das weiter vorstehende Folienband zwischen eine der Verschlussgliederreihen und das gegenüberliegende Band geschoben ist.

Das Folienband dichtet im Bereich der Verschlußgliederkette diese ab und verhindert den Flüssigkeitsaustritt in die Kuppelglieder. Zur wirkungsvollen Abdichtung ist das Folienband ausgelegt, um im geschlossenen Zustand der Verschlußgliederreihen abzudecken. Das Folienband bildet eine Dichtung, die einen Kontakt von Wundflüssigkeit mit der Verschlussgliederkette verhindert.

In einer bevorzugten Ausgestaltung sind zwei Folienbänder zwischen dem Abstandspolster und dem Tragband angeordnet. Bevorzugt kommen die Folienbänder nicht direkt mit der Wunde in Berührung, sondern lediglich das Abstandspolster liegt auf der Wunde auf.

In einer besonders zweckmäßigen Ausgestaltung sind Perforationen in jedem der Tragbänder vorgesehen. Die Perforationen dienen dazu, den Bereich unter dem Verband ausreichend mit Sauerstoff zu versorgen. In einer bevorzugten Ausgestaltung sind die Perforationen bezogen auf die Breite des Verbands dichter zur Mitte des Verbandes angeordnet als sie vom äußeren Rand beabstandet sind. Die Perforationen liegen nahe dem zentralen Abstandspolster. Gerade die Belüftung im zentralen Bereich nahe den Verschlußgliederreihen erlaubt es, auch bei Verwendung der Folienbänder den Wundbereich ausreichend zu belüften. Zudem erhält der Verband eine bessere Querelastizität, die bei Auftreten von üblichen Wundödemen eine Belastung der heilenden Wunde vermindert.

In einer zweckmäßigen Ausgestaltung ist jedes der Tragebänder mit einer Klebeschicht versehen. Der Verband kann über die Klebeschicht auf die Haut geklebt werden. Die Klebeschicht ist bevorzugt ein Acrylkleber.

In einer bevorzugten Ausgestaltung besitzt eines der Folienbänder eine größere Breite als das entsprechend andere Folienband.

Eine bevorzugte Ausgestaltung des erfindungsgemäßen Verbandes wird nachfolgend anhand von zwei Figuren näher erläutert.

Es zeigt:
- Fig. 1: eine teilweise geschnittene Darstellung zum Aufbau des Verbandes im geöffneten Zustand und
- Fig. 2: eine perspektivische Ansicht von schräg vorne für den Verband im geschlossenen Zustand.

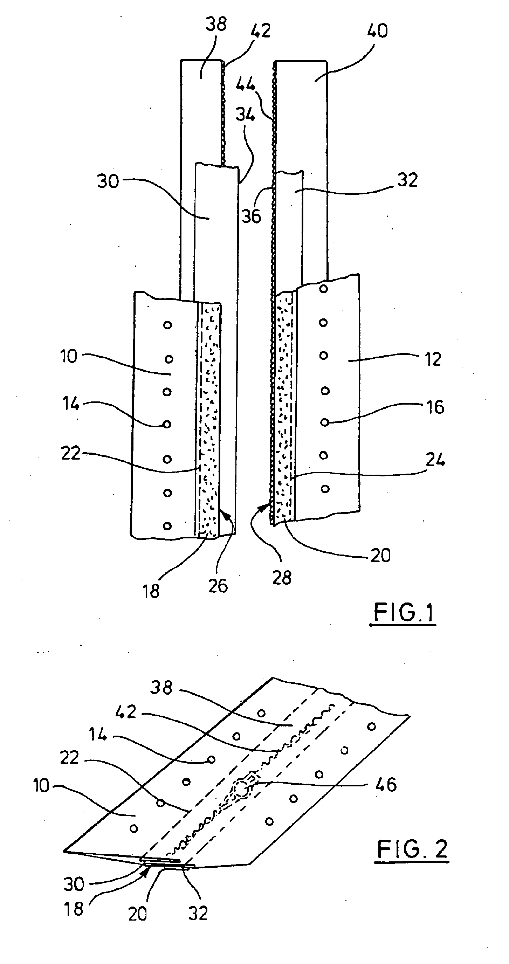

Figur 1 zeigt zwei Tragbänder 10, 12 die jeweils mit einer fortlaufenden Perforation 14, 16 versehen sind. Die Tragbänder 10, 12 sind auf der in Figur 1 dargestellten zugewandten Seite mit einer Klebeschicht aus Acrylkleber versehen, mit der das Tragband auf die Haut aufgeklebt werden kann. Im zentralen Bereich besitzen die Tragebänder ein Abstandspolster 18, 20, das zugleich auch als absorbierende Wundauflage dient. Das Abstandspolster 18, 20 ist an seinem nach außen weisenden Rand an dem Tragband 10 oder 12 befestigt. In Figur 1 und 2 erfolgt die Befestigung des Abstandspolsters über eine Naht 22, 24. Alternativ ist auch ein Verkleben mit beispielsweise einem Dreikomponentenkleber oder ein Verschweißen mit Ultraschall möglich. Die Kanten 26, 28 der Abstandspolster 18, 20 sind frei und nicht an einer Unterlage befestigt.

Unter den Abstandspolstern 18, 20 ist jeweils ein feuchtigkeitsundurchlässiges Folienband 30, 32 vorgesehen. Das Folienband ist feuchtigkeitsundurchlässig und besteht aus einem Kunststoffmaterial, bevorzugt PE und/oder PP. Die Folienbänder überlappen sich an ihren nach innen weisenden Kanten 34, 36. Wie deutlich in Figur 1 zu erkennen, steht das Folienband 30 weiter über das Abstandspolster 18 vor als das gegenüberliegende Folienband 32. Das vorstehende Ende des Folienbandes 30 bildet mit dem zweiten Folienband 32 eine überlappende Abdichtung für die Abstandspolster 18, 20. Das weiter vorstehende Folienband 30 schiebt sich dabei zwischen Reißverschluß und das bündig abschließende Folienband 32.

Die Folienbänder 30, 32 sind jeweils an den Tragbändern einer Verschlußgliederkette 38, 40 befestigt. Die Befestigung kann beispielsweise ebenfalls durch die Nähte 22, 24 erfolgen. Es kann aber auch eine andere Befestigung für die Folienbänder 30, 32 vorgesehen sein. Die Bänder 38 und 40 werden über zwei Verschlußgliederreihen 42 und 44 miteinander verbunden. Zum Verbinden der Verschlussgliederreihen 42 und 44 kann der in Figur 2 dargestellte Schieber 46 eingesetzt werden. Der Schieber 46 ist in der Reißverschlußwunde arretierbar und ist bevorzugt lackiert, um allergische Hautreaktionen (Kontaktallergie durch Nickel) zu vermeiden.

Figur 2 zeigt die Funktionsweise der Folienbänder 30, 32. Deutlich sichtbar ist, daß die Folienbänder unter den Abstandspolstern 18, 20 einander überlappen und so sicherstellen, daß eine aus der Wunde austretende Flüssigkeit nicht den Verschlußmechanismus für die Tragbänder verhindert. Die erfindungsgemäße Vorrichtung erfüllt zwei Aufgaben. Einerseits dient sie als ein Wundverschluß und zur Abdeckung der Wunde andererseits. Auch ist es möglich, die Perforationen 14, 16 näher an den zentralen Bereich des Verbandes heranzubringen, um so eine bessere Entlastung und Ventilation der Wunde zu erzielen. Das dargestellte Beispiel zeigt als Verschluß der beiden Tragbänder einen Reißverschluß. Es ist jedoch auch möglich, andere Formen eines wiederverschließbaren Verschlusses einzusetzen, wie beispielsweise einen Gleitverschluß oder einen Klettverschluß.

Die Länge des Reißverschlusses wird durch einen variabel anbringbaren Bottomdown-Stopp erreicht. Dieser kann beispielsweise aus einem schmalen Klebestreifen, beispielsweise aus dem gleichen Material wie das Tragband besteht.

## Patentansprüche

1. Vorrichtung zur atraumatischen Versorgung von Wunden, insbesondere zum Wundverschluß und zur Wundabdeckung, mit:
- einem Paar von Verschlußgliederreihen (42, 44), die ineinandergreifend jeweils an einem Tragband (10, 12) befestigt sind und mit Hilfe eines Schiebers (46) miteinander verbindbar und voneinander lösbar sind,
- mindestens einem an dem Tragband (10, 12) befestigten Abstandspolster (18, 20), das zur Auflage auf die Wunde vorgesehen ist, um Wundsekret zu absorbieren, und
- an jedem Tragband (10, 12) zwischen dem Abstandspolster (18, 20) und der Verschlussgliederreihe (42, 44) jeweils ein flüssigkeitsundurchlässiges Folienband (30, 32) vorgesehen ist,
**dadurch gekennzeichnet, daß**
- die Folienbänder (30, 32) im geschlossenen Zustand der Verschluss gliederreihen (42, 44) diese abdecken und einander überlappen,
- wobei ein Folienband (30) weiter über das Abstandspolster (18) vorsteht als das gegenüberliegende Folienband (32) und die Folienbänder eine überlappende Abdichtung bilden, bei der das weiter vorstehende Folienband (30) zwischen eine der Verschlussgliederreihen (44) und das gegenüberliegende Folienband (32) geschoben ist.

2. Vorrichtung nach Anspruch 1, **dadurch gekennzeichnet, daß** die Folienbänder (30, 32) zwischen Abstandspolster (18, 20) und Tragband (10, 12) angeordnet sind.

3. Vorrichtung nach einem der Ansprüche 1 oder 2, **dadurch gekennzeichnet, daß** jedes Tragband (10, 12) Perforationen (14, 16) aufweist.

4. Vorrichtung nach Anspruch 3, **dadurch gekennzeichnet, daß** die Perforation (14, 16) bezogen auf die Breite des Verbandes näher zur Mitte als zum äußeren Rand angeordnet sind.

5. Vorrichtung nach einem der Ansprüche 1 bis 4, **dadurch gekennzeichnet, daß** jedes Tragband (10, 12) mit einer hypoallergenen Klebeschicht versehen ist.

6. Vorrichtung nach einem der Ansprüche 1 bis 5, **dadurch gekennzeichnet, daß** eines der Folienbänder (30) eine größere Breite als das andere Folienband (32) besitzt.

## Claims

1. Device for non-traumatic caring for wounds, in particular for closing and covering wounds, with:
• a pair of closing element rows (42, 44), which are each one fastened on a support tape (10, 12) so as to mesh with each other, and adapted to be connected to each other and to be detached from each other by way of a slide (49),
• at least one spacer cushion (18, 20) fastened on the support tape (10, 12) and being provided for putting it onto the wound in order to absorb ichor, and
• one liquid-impermeable film tape (30, 32) being provided on each support tape (10, 12) between the spacer cushion (18, 20) and the closing element row (42, 44),
**characterised in that**
• in the closed condition of the closing element rows (42, 44), the film tapes (30, 32) cover the former and overlap with each other,
• wherein one film tape (30) projects farther over the spacer cushion (18) than the film tape (32) opposite to it, and the film tapes form an overlapping seal, in which the farther projecting film tape (30) is thrust between one of the closing element rows (44) and the opposite film tape (32).

2. Device according to claim 1, **characterised in that** the film tapes (30, 32) are disposed between spacer cushion (18, 20) and support tape (10, 12).

3. Device according to any one of claims 1 or 2, **characterised in that** each support tape (10, 12) has perforations (14, 16).

4. Device according to claim 3, **characterised in that** the perforations (14, 16) are disposed nearer to the centre than to the outer edge with respect to the width of the bandage.

5. Device according to any one of claims 1 to 4, **characterised in that** each support tape (10, 12) is provided with a hypoallergenic adhesive layer.

6. Device according to any one of claims 1 to 5, **characterised in that** one of the film tapes (30) has a greater width than the other film tape (32).

## Revendications

1. Dispositif d'alimentation atraumatique de plaies, notamment pour la fermeture de plaies et pour recouvrer des plaies, avec :
• une paire de lignes d'éléments de fermeture (42, 44), qui sont chacune fixé de façon engrenante sur une bande de support (10, 12) et adaptées à être reliées l'une à l'autre et détachées l'une de l'autre à l'aide d' un curseur (49),
• au moins un coussin écarteur (18, 20) fixé sur la bande de support (10, 12) et étant pourvu pour être mis sur la plaie afin d'absorber l'exsudat de la plaie, et
• un ruban de feuille (30, 32) imperméable pour liquide étant pourvu dans chaque bande de support (10, 12) entre le coussin écarteur (18, 20) et la ligne d'éléments de fermeture (42, 44)
**caractérisé en ce que**
• dans la condition fermée des lignes d'éléments de fermeture (42, 44), les rubans de feuille (30, 32) recouvrent celles-ci et se recouvrent eux-mêmes de façon chevauchante,
• un ruban de feuille (30) se projette plus loin au-dessus du coussin écarteur (18) que le ruban de feuille (32) vis-à-vis, et que les rubans de feuille forment un étanchement chevauchant, dans lequel le ruban de feuille (30) se projetant plus loin est poussé entre une des lignes d'éléments de fermeture (44) et le ruban de feuille (32) vis-à-vis.

2. Dispositif selon la revendication 1, **caractérisé en ce que** les rubans de feuille (30, 32) sont disposés entre le coussin écarteur (18, 20) et la bande de support (10, 12).

3. Dispositif selon l'une quelconque des revendications 1 ou 2, **caractérisé en ce que** chaque bande de support (10, 12) a des perforations (14, 16).

4. Dispositif selon la revendication 3, **caractérisé en ce que** les perforations (14, 16) sont disposés plus proche au centre qu'au bord extérieur par rapport à la largeur du pansement.

5. Dispositif selon l'une quelconque des revendications 1 à 4, **caractérisé en ce que** chaque bande de support (10, 12) est pourvue d'une couche adhésive hypoallergénique.

6. Dispositif selon l'une quelconque des revendications 1 à 5, **caractérisé en ce que** un des rubans de feuille (30) a une largeur plus grand que l'autre ruban de feuille (32).
